## Europäisches Patentamt
## European Patent Office
## Office européen des brevets

(19)

(11) Veröffentlichungsnummer : **0 152 809**
**B1**

(12) **EUROPÄISCHE PATENTSCHRIFT**

(45) Veröffentlichungstag der Patentschrift :
03.01.90

(51) Int. Cl.$^5$ : **B 01 J 23/80**, C 07 C 29/15

(21) Anmeldenummer : 85100811.0

(22) Anmeldetag : 26.01.85

(54) Katalysator zur Synthese von Methanol und höhere Alkohole enthaltenden Alkoholgemischen.

(30) Priorität : 02.02.84 DE 3403491

(43) Veröffentlichungstag der Anmeldung :
28.08.85 Patentblatt 85/35

(45) Bekanntmachung des Hinweises auf die Patenterteilung : 03.01.90 Patentblatt 90/01

(84) Benannte Vertragsstaaten :
AT BE CH DE FR GB IT LI LU NL SE

(56) Entgegenhaltungen :
EP-A- 0 125 689
DE-A- 3 005 551
US-A- 4 111 847
THE CANADIAN JOURNAL OF CHEMICAL ENGINEE-RING, Band 61, Februar 1983, Ottowa, Ontario K.J. SMITH AND R.B. ANDERSON "The Higher Alcohol synthesis Over Promoted Cu/ZnO Catalysts"

(73) Patentinhaber : SÜD-CHEMIE AG
Lenbachplatz 6
D-8000 München 2 (DE)

(72) Erfinder : Schneider, Michael, Dr.
Waldparkstrasse 54a
D-8012 Ottobrunn-Riemerling (DE)
Erfinder : Kochloefl, Karl, Dr.
Kreuzstrasse 2
D 8052 Moosburg (DE)
Erfinder : Bock, Ortwin
Im Wiesental 12
D-8300 Landshut-Kumhausen (DE)

(74) Vertreter : Reitzner, Bruno, Dr. et al
Patentanwälte Dipl.-Ing. R. Splanemann Dr. B. Reitzner Tal 13
D-8000 München 2 (DE)

EP 0 152 809 B1

**Beschreibung**

Die Erfindung betrifft einen Katalysator zur Synthese von Methanol und höhere Alkohole enthaltenden Alkoholgemischen aus CO und $H_2$, der in Form eines oxidischen Vorläufers

a) Kupferoxid und Zinkoxid, die durch Reduktion wenigstens eines Teils des Kupferoxids in die katalytisch wirksame Komponente überführbar sind,
b) Aluminiumoxid als thermostabilisierende Substanz und
c) mindestens ein Alkalicarbonat bzw. Alkalioxid enthält.

Derartige Katalysatoren sind beispielsweise aus der DE-A-30 05 551 bekannt. Bei diesen Katalysatoren wird durch einen Zusatz von Aklalioxiden, insbesondere von Kaliumoxid, die Bildung höherer Alkohole begünstigt. Alkoholgemische, bestehend aus Methanol und höheren aliphatischen Alkoholen, insbesondere mit Propanolen und Butanolen können als Treibstoff allein oder im Verschnitt mit Benzin zum Betrieb von Otto-Motoren eingesetzt werden. Werden derartige Gemische mit Benzin vermischt, so erfolgt aufgrund der Anwesenheit der höheren Alkohole keine Phasentrennung, wenn zufällig Wasser in kleiner Menge in das Benzin kommt bzw. wenn das Alkoholgemisch eine kleine Menge Wasser enthält.

Der Machanismus, nach welchem die höheren Alkohole neben dem Methanol gebildet werden, ist noch nicht genau bekannt. Man nimmt an, daß durch die Reaktion zwischen CO und $H_2$ an der Katalysatoroberfläche $-CH_2OH-$ Gruppen gebildet werden. Diese Oberflächengruppen werden durch das Alkali (A) in die Methylat-Oberflächengruppen $-CH_2OA$ übergeführt, an welche CO unter Bildung von Acetat-Oberflächengruppen $-CH_2-CO-OA$ angelagert wird.

Diese Gruppen werden mit Wasserstoff zu $-CH_2-CH_2-OH-$Oberflächenspezies reduziert. Diese Spezies bilden einerseits mit Wasserstoff Äthanol, andererseits mit Alkali die Äthylat-Oberflächengruppen $-CH_2-CH_2-OA$, die analog den Methylat-Oberflächengruppen durch Anlagerung von CO in höhere Alkohole übergeführt werden. Wahrscheinlich spielen auch Aldehyde bei der Synthese der höheren Alkohole eine Rolle, wodurch sich die Bildung von höheren aliphatischen Alkoholen mit verzweigter Kohlenstoffkette, wie zum Beispiel von i-Butanol, erklären läßt.

Bei den Katalysatoren nach der DE-A-30 05 551 liegt das Maximum der Ausbeute an höheren Alkoholen bei einem Gehalt an Kalium (berechnet als $K_2O$) von 1,7 Gew.-%. Der bevorzugte Bereich liegt zwischen 1,7 und 2,5 Gew.-%, wobei das Atomverhältnis Cu/Zn vorzugsweise zwischen 0,4 und 1,9 liegt.

Durch die Alkalisierung werden nicht nur neue Aktivzentren geschaffen, die die Synthese von höheren Alkoholen ermöglichen, sondern es werden gleichzeitig auch die Aktivzentren für die Methanolbildung blockiert. Der ansteigende Alkaligehalt im Katalysator hat demgemäß eine Abnahme der Methanolausbeute zur Folge.

Es wurde jedoch festgestellt, daß die Alkalisierung von Kupferoxid und Zinkoxid und Zinkoxid enthaltenden Methanolsynthesekatalysatoren zu einem schnelleren Wachstum der Cu-Kristallite und dadurch zu einer allmählichen Desaktivierung führt. Diese nachteilige Wirkung ist umso ausgeprägter, je stärker der Methanolsynthesekatalysator alkalisiert ist.

Ferner ist aus der US-A-4 111 847 ein Kupferoxid-Zinkoxid-Katalysator zur Synthese von Methanol bekannt, der durch Mischfällung von Zink- und Kupfercarbonat in einer Suspension von feinverteilten Aluminiumhydroxidteilchen, Trocknen und Calcinieren erhältlich ist.

Der Erfindung liegt die Aufgabe zugrunde, Katalysatoren der eingangs definierten Gattung zur Verfügung zu stellen, mit deren Hilfe bei einem verhältnismäßig niedrigen Alkalioxidgehalt (bei dem das Wachstum der Cu-Kristallite noch nicht ausgeprägt ist) hohe Ausbeuten an höheren Alkoholen erzielt werden können.

Überraschenderweise wurde gefunden, daß nicht nur die Alkalisierung, sondern auch die Porosität des oxidischen Katalysator-Vorläufers eine entscheidende Rolle bei der Synthese von höheren Alkoholen aus CO und $H_2$ spielt. Es wurde festgestellt, daß die Ausbeute an höheren Alkoholen dem Volumen der Poren mit einem Durchmesser von weniger als etwa 14 nm im allgemeinen proportional ist. Gegenstand der Erfindung ist daher ein Katalysator der eingangs definierten Gattung, der dadurch gekennzeichnet ist, daß der oxidische Vorläufer einen Anteil an Poren mit einem Durchmesser zwischen 14 und 7,5 nm von 20 bis 70 % des Gesamtvolumens besitzt (nach der Methode der Quecksilberporosimetrie bestimmt), der Alkaligehalt etwa 13 — $130 \times 10^{-6}$ Grammatom Alkalimetall je Gramm des oxidischen Vorläufers beträgt, die Aluminiumoxidkomponente aus einem kolloidal verteilten Aluminiumhydroxid (Aluminiumhydroxid- Sol oder -Gel) erhalten worden ist und die Kupfer- und Zinkoxid-Komponente (a) durch Zusatz einer alkalisch reagierenden Substanz aus einer Lösung der entsprechenden Salze in Gegenwart des kolloidal verteilten Aluminiumhydroxids ausgefällt worden ist.

Man nimmt an, daß sich die Alkalioxide bzw. -carbonate bevorzugt im Inneren der Poren befinden. Der Anteil an Poren mit einem Durchmesser zwischen 14 und 7,5 nm beträgt vorzugsweise 25 bis 55 % des gesamten Porenvolumens. Der Mechanismus, nach welchem das kolloidal verteilte Aluminiumhydroxid den Porendurchmesser beeinflußt, ist nicht bekannt. Die Teilchengröße des kolloidal verteilten Aluminiumhydroxids ist nicht besonders kritisch. Kolloidal verteilte Aluminiumhydroxide in Form der entsprechenden Sole oder Gele sind im Handel erhältlich, können aber z. B. auch durch Peptisierung des

2

frisch hergestellten Aluminiummethahydrats (Al(OH)O) mit verdünnter Salpetersäure gewonnen werden. Unter dem Begriff « Aluminiumhydroxid » sollen erfindungsgemäß alle hydratisierten Formen des Aluminumoxids unabhängig vom Grad der Hydratisierung fallen, vorausgesetzt, daß die Teilchen in kolloidaler Verteilung vorliegen. Im allgemeinen werden die Aluminiumhydrate durch die Formel $(AL_2O_3 \cdot nH_2O)$ erfaßt, wobei n zwischen etwa 0,1 und 3 liegt und im allgemeinen auch höher sein kann, wenn man auch die adsorptiv gebundenen Wassermoleküle der Hydrathülle berücksichtigt.

Der Porendurchmesser wird nach der Methode der Quecksilberporosimetrie bestimmt (vgl. z. B. R. Anderson, Experimental Methods in Catalytic Research, Academic Press, New York, 1968). Nach dieser Methode wird Quecksilber auf den geformten, oxidischen Katalysator-Vorläufer aufgepreßt. Der Druck (P), der zur Überwindung der Kapillardepression in den Poren des Vorläufers erforderlich ist, ist nach der Washburn-Gleichung umgekehrt proportional dem Porendurchmesser (d) :

$$d = \frac{2\,\gamma\,\cos\theta_c}{P}$$

($\gamma$ ist eine Proportionalitätskonstante ; $_c$ ist der Kontaktwinkel des Quecksilbers).

Der erfinderische Katalysator ist in Form seines oxidischen Vorläufers definiert, da es zweckmäßiger ist, den Porendurchmesser an diesem zu bestimmen, weil der durch Reduktion aktivierte Katalysator gegenüber dem Luftsauerstoff empfindlich ist. Die Porenverteilung ändert sich nach der Reduktion nur unwesentlich.

Der erfindungsgemäße Katalysator hat im allgemeinen ein Atomverhältnis Cu/Zn von 1,3 bis 3,8 : 1, vorzugsweise von 2,2 bis 2,9 : 1. Dieses Atomverhältnis ist im allgemeinen größer als das Atomverhältnis der Katalysatoren nach der DE-A-30 05 551, das im allgemeinen zwischen 0,4 bis 1,9 : 1 beträgt. Es kann also erfindungsgemäß der Anteil der Kupferkomponente erhöht werden, wodurch eine Aktivitätssteigerung erzielt wird. Da aufgrund der erfindungsgemäßen Porenstruktur der Alkaligehalt niedriger sein kann, ist die Bildung von Kupferkristalliten, die bei einem höheren Atomverhältnis Cu/Zn an sich begünstigt ist, herabgesetzt.

Der erfindungsgemäße Katalysator enthält im allgemeinen 5 bis 25 Gew.-%, vorzugsweise 14 bis 18 Gew.-% Aluminiumoxid (bezogen auf den oxidischen Vorläufer). Das Aluminiumoxid wird zumindest zum größten Teil in Form des kolloidal verteilten Aluminiumhydroxids eingeführt. Das Aluminiumoxid bewirkt eine erhöhte Thermoresistenz des Katalysators.

Der erfindungsgemäße Katalysator enthält als Alkalimetalle vorzugsweise Kalium, Rubidium und/oder Cäsium, und zwar in Mengen von etwa $13\text{-}130 \times 10^{-6}$, vorzugsweise von $50\text{-}100 \times 10^{-6}$ Grammatom je Gramm des oxidischen Vorläufers. Diese Mengen bewirken zwar aufgrund der bevorzugten Porenstruktur des erfindungsgemäßen Katalysators eine Erhöhung der Ausbeute an höheren Alkoholen, sind aber so gering, daß Änderungen der physikalischen Eigenschaften des Katalysators, z. B. das vorstehend bereits erwähnte Anwachsen von Cu-Kristalliten und sonstige Änderungen der Oberflächenstruktur und der Porosität wesentlich geringer sind als bei den Katalysatoren nach dem Stand der Technik.

Als weitere thermostabilisierende Substanzen neben dem Aluminiumoxid kann der erfindungsgemäße Katalysator bis zu 10 Gew.-%, vorzugsweise 3 bis 7 Gew.-% $CeO_2$ und/oder $La_2O_3$ (bezogen auf den oxidischen Vorläufer) enthalten.

Die erfindungsgemäßen Katalysatoren sind im allgemeinen dadurch erhältlich, daß man den die Kupfer- und Zinkoxid-Komponente (a) und das kolloidal verteilte Aluminiumhydroxid enthaltenden Niederschlag wäscht, gegebenenfalls calciniert, mit Verbindungen der Alkalimetalle imprägniert, trocknet und in an sich bekannter Weise zu Formkörpern verpreßt.

Im allgemeinen verwendet man Lösungen der Nitrate von Cu und Zn sowie gegebenenfalls von Ce und La und führt die Fällung vorzugsweise mit einer wäßrigen $K_2CO_3$-Lösung durch. Die Konzentration der Lösung beträgt vorzugsweise 5 bis 20 Gew.-%.

Statt von den Nitraten kann man aber auch von den entsprechenden Metallformiaten oder -acetaten ausgehen. Die Fällung kann auch mit Hilfe einer Kaliumbicarbonat-Lösung durchgeführt werden. Führt man die Fällung mit $K_2CO_3$ bzw. $KHCO_3$ durch, so braucht man den Niederschlag nicht besonders gründlich auszuwaschen, da bei der anschließenden Alkalisierung ohnehin bevorzugter Kalium verwendet wird. Aus diesem Grund kann man die Fällung natürlich auch mit Rubidium- oder Cäsiumcarbonat bzw. -bicarbonat durchführen. Da jedoch ein großer Teil dieser Kationen beim Auswaschen entfernt wird, zieht man es vor, von dem billigeren Kalium-carbonat bzw. -bicarbonat auszugehen. Ferner kann die Fällung mit Natriumcarbonat bzw. Natriumbicarbonat durchgeführt werden ; bei dieser Methode muß jedoch der Katalysator-Vorläufer anschließend verhältnismäßig gründlich ausgewaschen werden. Die Fällung ist auch mit Ammoniumcarbonat bzw. -bicarbonat möglich.

Die Fällung wird im allgemeinen bei Temperaturen von 20 bis 65 °C und bei pH-Werten im Bereich von 6,5 bis 7,5 durchgeführt. Im allgemeinen arbeitet man bei Raumtemperatur (25 °C) und bei einem konstanten pH-Wert von 6,9 ± 0,1.

Die Fällung kann ansatzweise oder kontinuierlich durchgeführt werden. Vorzugsweise führt man die Fällung durch kontinuierliche Zusammenführung der das kolloidal verteilte Aluminiumhydroxid enthaltenden Lösung der Nitrate von Cu und Zn sowie gegebenenfalls von Ce und La mit wäßriger $K_2CO_3$-

Lösung durch.

Anschließend an die Fällung wird der gewaschene Niederschlag des oxidischen Katalysatorvorläufers vorzugsweise bei etwa 270 bis 290 °C calciniert, gegebenenfalls zerkleinert (im allgemeinen auf < 1,0 mm) und durch Behandlung mit einer Lösung der Alkalimetallverbindung(en), vorzugsweise unter vermindertem Druck, alkalisiert. Zu diesem Zweck kann man ein den Katalysatorvorläufer enthaltendes Gefäß auf etwa 10 bis 50 Torr (13,3.10⁻³ bis 66,7.10⁻³ bar) evakuieren und die Lösung der Alkalimetallverbindungen in das Gefäß einführen. Zur Alkalisierung des oxidischen Vorläufers verwendet man vorzugsweise die Hydroxide, Carbonate, Hydrogencarbonate, Formiate und/oder Acetate des Kaliums, Rubidiums und/oder Cäsiums. Im allgemeinen werden zu diesem Zweck wäßrig-alkoholische Lösungen verwendet, obwohl man auch wäßrige Lösungen verwenden kann. Bevorzugt verwendet man wäßrig-methanolische oder wäßrig-äthanolische Lösungen.

Die alkalisierten Katalysatorvorläufer werden nach dem Trocknen im allgemeinen in an sich bekannter Weise zu Formkörpern verpreßt, beispielsweise zu Tabletten von 4,5 × 3 mm bzw. von 6 × 3 mm, wobei Gleitmittel, wie Graphit, zugesetzt werden können.

Der oxidische Katalysator-Vorläufer wird üblicherweise dadurch aktiviert, daß er einer reduzierenden Nachbehandlung unterzogen wird. Diese kann unmittelbar im Synthesereaktor erfolgen und wird vorzugsweise so durchgeführt, daß zunächst mit Hilfe eines eine kleine Menge Wasserstoff enthaltenden inerten Gases, wie Stickstoff, reduziert wird. Der Stickstoff enthält üblicherweise zunächst etwa 1,5 Vol.-% $H_2$. Hierbei wird die Temperatur beispielsweise von 100 auf 235 °C über einen Zeitraum von 16 Stunden angehoben. Danach wird der Wasserstoffanteil erhöht, wobei z. B. mit 20 Vol.-% $H_2$ (Rest $N_2$) über einen Zeitraum von 3 Stunden im Temperaturbereich von 235 bis 270 °C gearbeitet werden kann. Die Vollendung der reduktiven Behandlung kann mit 99,9 %igem $H_2$ über einen Zeitraum von 3 Stunden bei 270° bis 300 °C erfolgen. Üblicherweise wird mit einer Raumgeschwindigkeit von etwa 1 000 bis 2 000 Liter Reduktionsgas pro Stunde und Liter Katalysator aktiviert.

Gegenstand der Erfindung ist ferner die Verwendung der vorstehend beschriebenen Katalysatoren zur Synthese von Methanol und höhere Alkohole enthaltenden Alkoholgemischen aus CO und $H_2$. Die Synthese wird üblicherweise bei einer Temperatur von etwa 250 bis 320 °C, vorzugsweise bei 280 bis 300 °C, bei einem Druck von etwa 80 bis 150 bar, vorzugsweise bei etwa 100 bar, und bei einer Raumgeschwindigkeit von etwa 1 000 bis 10 000, vorzugsweise von 3 000 bis 5 000 Liter Synthesegas pro Stunde und Liter Katalysator durchgeführt, wobei das Synthesegas etwa 25 bis 60, vorzugsweise 30 bis 50 Vol.-% CO, etwa 0 bis 4 Vol.-% $CO_2$, etwa 0 bis 4 Vol.-% $N_2$ bzw. $CH_4$ und als Rest $H_2$ enthalten kann.

Die Herstellung und Anwendung der Katalysatoren ist durch die nachstehenden Beispiele erläutert.

Beispiel 1 (Vergleichsbeispiel)

Zur Herstellung des Katalysatorvorläufers (Vergleichskatalysator) wurde wie folgt verfahren :

Als Carbonatlösung diente eine Lösung von 12 Gew.-% $K_2CO_3$ in deionisiertem Wasser. Zur Herstellung der Metallnitratlösung wurden 626,4 g $Cu(NO_3)_2 \cdot 3H_2O$, 241,0 g $Zn(NO_3)_2 \cdot 4H_2O$ und 256,1 g $Al(NO_3)_3 \cdot 9H_2O$ in 2 Liter deionisiertem Wasser gelöst und auf 4 Liter verdünnt.

Die kontinuierliche Fällung erfolgte in einer Fällapparatur, bestehend aus einem mit einem Rührwerk versehenen, 600 ml fassendem Fällgefäß und einem 25 Liter fassenden Auffanggefäß. Im Fällgefäß wurden zunächst 400 ml deionisiertes Wasser vorgelegt, etwas Metallnitratlösung zugegeben und mit Carbonatlösung auf einen pH-Wert von 6,9 ± 0,1 eingestellt. Unter ständigem Rühren wurde nun der gleichzeitige Zulauf von Metallnitrat- und Carbonatlösung so geregelt, daß im Fällgefäß der pH-Wert von 6,9 ± 0,1 aufrechterhalten wurde. Die Fällung erfolgte bei 25 °C. Die Fällzeit betrug 15 bis 20 Minuten.

Die erhaltene Suspension wurde im Auffanggefäß noch 30 Min. bei Raumtemperatur gerührt, dann über eine Nutsche abfiltriert und durch mehrfaches Aufschlämmen mit jeweils 4 Liter deionisiertem Wasser von 50 °C gewaschen. Der Filterkuchen wurde danach bei 120 °C getrocknet und in dünner Schicht 8 Stunden bei 280 °C calciniert. Das calcinierte Produkt enthielt 228 ppm K (durch Atomabsorptions-spektrometrie bestimmt).

200 g des calcinierten und granulierten (< 1 mm) Katalysatorvorläufers wurden unter gutem Durchmischen mit einer Lösung von 800 mg $K_2CO_3$ in 10 ml $H_2O$ und 20 ml Methanol besprüht und dann für 30 Min. dem Vakuum einer Wasserstrahlpumpe ausgesetzt. Das imprägnierte Produkt wurde bei 120 °C getrocknet.

Nach dem Zumischen von 2 % Naturgraphit als Gleitmittel wurde das Produkt zu Tabletten mit einem Durchmesser von 6 mm und einer Höhe von 3 mm verpreßt. Tabelle I zeigt die chemischen und Tabelle II die physikalisch-mechanischen Daten des so erhaltenen Katalysators 1.

Beispiel 2

Herstellung eines erfindungsgemäßen Katalysatorvorläufers

Als Carbonatlösung wurde eine Lösung von 12 Gew.-% $K_2CO_3$ in deionisiertem Wasser verwendet. Zur Herstellung der Metallnitratlösung wurden 1 253 g $Cu(NO_3)_2 \cdot 3H_2O$ und 482 g $Zn(NO_3)_2 \cdot 4H_2O$ in 4 Liter deionisiertem Wasser gelöst, worauf 696 g Aluminiumhydroxidsol (mit 10 Gew.-% $Al_2O_3$) zugegeben

und mit deionisiertem Wasser auf 8 Liter verdünnt wurden.

Die kontinuierliche Fällung, das Auswaschen und die Calcinierung erfolgten analog Beispiel 1.

Der calcinierte Katalysatorvorläufer enthielt 260 ppm K.

200 g des granulierten (< 1 mm) Katalysatorvorläufers wurden unter gutem Durchmischen mit einer Lösung von 800 mg $K_2CO_3$ in 10 ml $H_2O$ und 20 ml Methanol besprüht und dann 30 Min. dem Vakuum einer Wasserstrahlpumpe ausgesetzt. Dann wurde das Produkt bei 120 °C getrocknet.

Nach dem Zumischen von 2% Naturgraphit als Gleitmittel wurde das Produkt zu Tabletten mit einem Durchmesser von 6 mm und einer Höhe von 3 mm verpreßt. Tabelle I enthält die chemischen, Tabelle II die physikalisch-mechanischen Daten des so erhaltenen Katalysators 2.

## Beispiel 3

Herstellung eines erfindungsgemäßen Katalysatorvorläufers

200 g des im Beispiel 2 beschriebenen, granulierten Katalysator-Vorläufers wurden unter gutem Durchmischen mit einer Lösung von 1 480 mg $Rb_2CO_3$ in 10 ml $H_2O$ und 20 ml Methanol besprüht und dann 30 Min. dem Vakuum einer Wasserstrahlpumpe ausgesetzt. Das Produkt wurde dann bei 120 °C getrocknet.

Nach dem Zumischen von 2 % Naturgraphit wurde das Produkt zu Tabletten mit einem Durchmesser von 6 mm und einer Höhe von 3 mm verpreßt.

Tabelle I enthält die chemischen und Tabelle II die physikalisch-mechanischen Daten des Katalysators 3.

## Beispiel 4

Herstellung eines erfindungsgemäßen Katalysatorvorläufers

200 g des im Beispiel 2 beschriebenen, granulierten Katalysator-Vorläufers wurden unter gutem Durchmischen mit einer Lösung von 2 085 mg $Cs_2CO_3$ in 10 ml $H_2O$ und 20 ml Methanol besprüht und dann 30 Min. dem Vakuum einer Wasserstrahlpumpe ausgesetzt. Dann wurde das Produkt bei 120 °C getrocknet.

Nach dem Zumischen von 2 % Naturgraphit wurde das Produkt zu Tabletten mit einem Durchmesser von 6 mm und einer Höhe von 3 mm verpreßt.

Tabelle I enthält die chemischen und Tabelle II die physikalisch-mechanischen Daten des so erhaltenen Katalysators 4.

## Beispiel 5

Herstellung eines erfindungsgemäßen Katalysatorvorläufers

Als Carbonatlösung wurde eine Lösung von 12 Gew.-% $K_2CO_3$ in deionisiertem Wasser verwendet. Zur Herstellung der Metallnitratlösung wurden 626,4 g $Cu(NO_3)_2 \cdot 3H_2O$ und 241.0 g $Zn(NO_3)_2 \cdot 4H_2O$ in 2 Liter deionisiertem Wasser gelöst, worauf 617 g Aluminiumhydroxidsol (mit 10 Gew.-% $Al_2O_3$) zugegeben und mit deionisiertem Wasser auf 4 Liter verdünnt wurden.

Die kontinuierliche Fällung, das Auswaschen und die Calcinierung erfolgten analog Beispiel 1.

Der calcinierte Katalysatorvorläufer enthielt 315 ppm K.

200 g des Katalysatorvorläufers wurden mit einer Lösung von 1 300 mg $K_2CO_3$ in 30 ml $H_2O$ unter gutem Durchmischen besprüht und dann 30 Min. dem Vakuum einer Wasserstrahlpumpe ausgesetzt. Dann wurde bei 120 °C getrocknet. Nach dem Zumischen von 2 % Naturgraphit wurde das Produkt zu Tabletten mit einem Durchmesser von 6 mm und einer Höhe von 3 mm verpreßt.

Tabelle I enthält die chemischen und Tabelle II die physikalisch-mechanischen Daten des Katalysators 5.

## Beispiel 6

Herstellung eines erfindungsgemäßen Katalysatorvorläufers

Als Carbonatlösung wurde eine Lösung von 12 Gew.-% $K_2CO_3$ in deionisiertem Wasser verwendet. Zur Herstellung der Metallnitratlösung wurden 626,4 g $Cu(NO_3)_2 \cdot 3H_2O$, 241,0 g $Zn(NO_3)_2 \cdot 4H_2O$, 31,8 g $Ce(NO_3)_3 \cdot 6H_2O$ und 31,9 g $La(NO_3)_3 \cdot 6H_2O$ in 2 Liter deionisiertem Wasser gelöst, worauf 377 g Aluminiumhydroxidsol (mit 10 Gew.-% $Al_2O_3$) zugegeben und auf 4 Liter verdünnt wurden.

Die kontinuierliche Fällung, das Auswaschen und die Calcinierung erfolgten analog Beispiel 1.

Der calcinierte Katalysatorvorläufer enthielt 280 ppm K.

200 g des Katalysatorvorläufers wurden unter gutem Durchmischen mit einer Lösung von 800 mg $K_2CO_3$ in 10 ml $H_2O$ und 20 ml Äthanol besprüht und dann 30 Min. dem Vakuum einer Wasserstrahlpum-

**EP 0 152 809 B1**

pe ausgesetzt. Dann wurde das Produkt bei 120 °C getrocknet. Nach dem Zumischen von 2 % Naturgraphit wurde das Produkt zu Tabletten mit einem Durchmesser von 6 mm und einer Höhe von 3 mm verpreßt.

Tabelle I enthält die chemischen und Tabelle II die physikalisch-mechanischen Daten des so erhaltenen Katalysators 6.

(Siehe Tabellen Seite 7 ff.)

Tabelle I

| Katalysator | G V * 600°C Gew.-% | CuO Gew.-% | ZnO Gew.-% | Al₂O₃ Gew.-% | CeO₂ Gew.-% | La₂O₃ Gew.-% | K Gew.ppm | Rb Gew.ppm | Cs Gew.ppm |
|---|---|---|---|---|---|---|---|---|---|
| 1 | 13.5 | 56.4 | 20.5 | 9.5 | - | - | 2490 | - | - |
| 2 | 14.9 | 55.4 | 20.2 | 9.4 | - | - | 2520 | - | - |
| 3 | 14.8 | 55.4 | 20.2 | 9.4 | - | - | 262 | 5475 | - |
| 4 | 14.8 | 55.4 | 20.2 | 9.4 | - | - | 260 | - | 8505 |
| 5 | 12.7 | 52.5 | 19.1 | 15.7 | - | - | 3995 | - | - |
| 6 | 14.0 | 51.6 | 18.8 | 9.4 | 3.1 | 3.0 | 2540 | - | - |

* GV = Glühverlust

EP 0 152 809 B1

Tabelle II

| Kat. | S G (g/100 ml) | S D F (kg/Tabl.) | Os (m²/g) | PV (ml/g) | Porenverteilung (%) | | | |
| --- | --- | --- | --- | --- | --- | --- | --- | --- |
| | | | | | 15000–1750 (nm) | 1750 – 80 (nm) | 80 – 14 (nm) | 14 – 7.5 (nm) |
| 1 | 88.4 | 12.9 (10.5-15.9) | 98 | 0.41 | 0.5 | 13.6 | 75.3 | 11,1 |
| 2 | 93.0 | 13.7 (11.0-15.9) | 130 | 0,29 | 0,8 | 1,6 | 55,6 | 42,0 |
| 3 | 92.5 | 13.2 (10.1-14.8) | 129 | 0,29 | 0,2 | 0.9 | 55,4 | 43,5 |
| 4 | 93.0 | 13.4 (10.9-15.1) | 131 | 0,28 | 0.1 | 1,5 | 54,4 | 44,0 |
| 5 | 85.0 | 13.4 (12.5-14.5) | 130 | 0.45 | 0.2 | 2,3 | 47,5 | 50,0 |
| 6 | 86.4 | 13.8 (10.0-15.2) | 109 | 0.39 | 0.5 | 1.0 | 63.2 | 36,0 |

Anmerkungen : SG = Spez. Gewicht ; SDF = Seitendruckfestigkeit ; $O_s$ = spez. Oberfl. (nach BET).
PV = Porenvolumen

EP 0 152 809 B1

Beispiel 7

(Anwendungsbeispiel)

Je 30 cm³ der nach den Beispielen 1 bis 6 hergestellten Katalysatorvorläufer wurden in einem Durchflußreaktor (Autoclave Engineers, Erie, USA), mit einem internen Gaskreislauf, mit einem Gas, bestehend aus 1.5 Vol.-% $H_2$, Rest $N_2$, über einen Zeitraum von 16 Std. von 100 bis 235 °C aktiviert. Danach wurde die Aktivierung mit 20 Vol.-% $H_2$ (Rest $N_2$) über einen Zeitraum von 3 Stunden von 235 bis 270 °C fortgesetzt und schließlich über einen Zeitraum von 3 Stunden mit 99.9 %igem $H_2$ bei 270 bis 300 °C vollendet. Anschließend wurde Synthesegas Nr. 1 oder Nr. 2

|  | SG-1 | SG-2 |
|---|---|---|
| CO (Vol.%) | 29.0 | 50.0 |
| $CO_2$ " | 0,5 | 1.0 |
| $H_2$ " | Rest | Rest |

über einen Durchflußmesser dem Reaktor zugeführt, wobei ein Druck von 100 bar und eine Raumgeschwindigkeit von 4 000 Liter SG/Std. und Liter Katalysator eingestellt wurden. Die Reaktionsprodukte wurden nach dem Durchgang durch einen Wasserkühler (10 °C) in flüssige und gasförmige getrennt. Die flüssigen Reaktionsprodukte wurden in periodischen Abständen von 8 Stunden abgelassen, gewogen und mit Hilfe der Gas-Flüssigkeitschromatographie analysiert und mittels eines elektronischen Integrators quantitativ ausgewertet. Die gasförmigen Reaktionsprodukte wurden nach der kontinuierlichen Entspannung mit Hilfe eines Gasometers gemessen und gaschromatographisch analysiert. Die quantitative Auswertung geschah ebenfalls mit Hilfe eines elektronischen Integrators. Die Testergebnisse für die hergestellten Katalysatoren 1 bis 6 sind in der Tabelle III zusammengestellt.

(Siehe Tabelle Seite 10 f.)

EP 0 152 809 B1

Tabelle III

Ergebnisse der Synthese von Gemischen, bestehend aus Methanol und höheren Alkoholen T = 300 °C,
P = 100 bar Synthesegas, RG = 4 000 Liter SG pro Std. und Liter Kat.
Synthesegas Nr. 1 : CO 29.0 Vol % $CO_2$ 0.5 Vol %, $H_2$ Rest
Synthesegas Nr. 2 : CO 49 Vol % $CO_2$ 1.0 Vol %, $H_2$ Rest

| | | | | | | | | |
|---|---|---|---|---|---|---|---|---|
| Katalysator Nr. | 1 | 2 | 2 | 3 | 4 | 4 | 5 | 6 |
| Synthesegas Nr. | 1 | 1 | 2 | 1 | 1 | 2 | 1 | 1 |
| CO-Umsatz  Mol % | 49.2 | 59.0 | 31.3 | 58.5 | 56.1 | 29.6 | 57.2 | 56.1 |
| Ausbeute an flüssigen | 762 | 895 | 770 | 875 | 860 | 710 | 840 | 833 |
| Reaktionsprodukten (g/h $1_{Kat.}$) | | | | | | | | |
| Zusammensetzung Gew.-% | | | | | | | | |
| Methanol | 93.4 | 92.0 | 86.1 | 91.0 | 90.0 | 83.5 | 90.0 | 91.7 |
| Hoehere Alkohole | 4.1 | 5.4 | 9.3 | 6.4 | 7.0 | 11.2 | 6.2 | 5.7 |
| $H_2O$ | 1.4 | 1.5 | 2.6 | 1.6 | 1.8 | 3.1 | 1.8 | 1.1 |
| Nebenprodukte | 1.1 | 1.1 | 2.0 | 1.0 | 1.2 | 2.2 | 2.0 | 1.5 |
| Ausbeute an hoeheren Alkoholen (g/Std. Liter Kat.) | 31.2 | 48.3 | 71.6 | 56.0 | 60.2 | 79.5 | 52.1 | 47.5 |
| Zusammensetzung der höheren Alkohole Gew.-% | | | | | | | | |
| $C_2H_5OH$ | 35.4 | 31.6 | 27.3 | 26.3 | 23.0 | 19.9 | 21.1 | 33.0 |
| $C_3H_7OH$ | 20.2 | 20.9 | 18.9 | 19.1 | 16.7 | 15.8 | 17.5 | 21.2 |
| $C_4H_9OH$ | 20.3 | 21.5 | 22.1 | 27.7 | 29.2 | 30.5 | 31.8 | 22.2 |
| $C_5H_{11}OH$ | 10.1 | 10.4 | 9.5 | 11.9 | 13.4 | 12.2 | 12.5 | 9.6 |
| $>C_5H_{11}OH+$ andere org. Verbindungen | 14.0 | 15.6 | 22.2 | 15.0 | 17.7 | 21.6 | 17.1 | 14.0 |

EP 0 152 809 B1

## Patentansprüche

1. Katalysator zur Synthese von Methanol und höhere Alkohole enthaltenden Alkoholgemischen aus CO und $H_2$, der in Form eines oxidischen Vorläufers

a) Kupferoxid und Zinkoxid, die durch Reduktion wenigstens eines Teils des Kupferoxids in die katalytisch wirksame Komponente überführbar sind,

b) Aluminiumoxid als thermostabilisierende Substanz und

c) mindestens ein Alkalicarbonat bzw. Alkalioxid enthält, dadurch gekennzeichnet, daß der oxidische Vorläufer einen Anteil an Poren mit einem Durchmesser zwischen 14 und 7,5 nm von 20 bis 70 % des Gasamtvolumens besitzt, der Alkaligehalt etwa 13-$130 \times 10^{-6}$ Grammatom Alkalimetall je Gramm des oxidischen Vorläufers beträgt und die Aluminiumoxidkomponente aus einem kolloidal verteilten Aluminiumhydroxid (Aluminiumhydroxid-Sol oder -Gel) erhalten worden ist und die Kupfer- und Zinkoxid-Komponente (a) durch Zusatz einer alkalisch reagierenden Substanz aus einer Lösung der entsprechenden Salze in Gegenwart des kolloidal verteilten Aluminiumhydroxids ausgefällt worden ist.

2. Katalysator nach Anspruch 1, dadurch gekennzeichnet, daß der Anteil an Poren mit einem Durchmesser zwischen 14 und 7,5 nm 25 bis 55 % des gesamten Porenvolumens beträgt.

3. Katalysator nach Anspruch 1 oder 2, dadurch gekennzeichnet, daß das Atomverhältnis Cu/Zn 1,3 bis 3,8 : 1, vorzugsweise 2,2 bis 2,9 :1 beträgt.

4. Katalysator nach einem der Ansprüche 1 bis 3, dadurch gekennzeichnet, daß er 5 bis 25 Gew.-%, vorzugsweise 14 bis 18 Gew.-% Aluminiumoxid (bezogen auf den oxidischen Vorläufer) enthält.

5. Katalysator nach einem der Ansprüche 1 bis 4, dadurch gekennzeichnet, daß er 13-$130 \times 10^{-6}$ Grammatom, vorzugsweise 50-$100 \times 10^{-6}$ Grammatom Kalium, Rubidium und/oder Cäsium je Gramm des oxidischen Vorläufers enthält.

6. Katalysator nach einem der Ansprüche 1 bis 5, dadurch gekennzeichnet, daß er als weitere thermostabilisierende Substanzen bis zu 10 Gew.-%, vorzugsweise 3 bis 7 Gew.-% $CeO_2$ und/oder $La_2O_3$ (bezogen auf den oxidischen Vorläufer) enthält.

7. Katalysator nach einem der Ansprüche 1 bis 6, dadurch erhältlich, daß man den die Kupfer- und Zinkoxid-Komponente (a) und das kolloidal verteilte Aluminiumhydroxid enthaltenden Niederschlag wäscht, gegebenenfalls calciniert, mit Verbindunger der Alkalimetalle imprägniert, trocknet und in an sich bekannter Weise zu Formkörpern verpreßt.

8. Katalysator nach Anspruch 1, dadurch erhältlich, daß man Lösungen der Nitrate von Cu und Zn sowie gegebenenfalls von Ce und La verwendet und die Fällung mit einer wäßrigen $K_2CO_3$-Lösung mit einer Konzentration von vorzugsweise 5 bis 20 Gew.-% durchführt.

9. Katalysator nach Anspruch 7 oder 8, dadurch erhältlich, daß man die Fällung bei Temperaturen von 20 bis 65 °C und bei pH-Werten im Bereich von 6,5 bis 7,5 durchführt.

10. Katalysator nach einem der Ansprüche 7 bis 9, dadurch erhältlich, daß man die Fällung durch kontinuierliche Zusammenführung der das kolloidal verteilte Aluminiumhydroxid enthaltenden Lösung der Nitrate von Cu und Zn sowie gegebenenfalls von Ce und La mit wäßriger $K_2CO_3$-Lösung durchführt.

11. Katalysator nach einem der Ansprüche 7 bis 10, dadurch erhältlich, daß man den gewaschenen Niederschlag des oxidischen Vorläufersbei etwa 270 bis 290 °C calciniert, gegebenenfalls zerkleinert und durch Behandlung mit einer wäßrigen oder wäßrig-alkoholischen Lösung der Alkalimetallverbindung(en), vorzugsweise unter vermindertem Druck, alkalisiert.

12. Katalysator nach einem der Ansprüche 7 bis 11, dadurch erhältlich, daß man zur Alkalisierung des oxidischen Vorläufers die Hydroxide, Carbonate, Hydrogencarbonate, Formiate und/oder Acetate des Kaliums, Rubidiums und/oder Cäsiums verwendet.

13. Verwendung des Katalysators nach einem der Ansprüche 1 bis 12 zur Synthese von Methanol und höhere Alkohole enthaltenden Alkoholgemischen aus CO und $H_2$.

14. Verwendung nach Anspruch 13, dadurch gekennzeichnet, daß die Synthese bei einer Temperatur von etwa 250 bis 350 °C, bei einem Druck von 80 bis 150 bar und bei einer Raumgeschwindigkeit von etwa 1 000 bis 10 000 Liter Synthesegas pro Stunde und Liter Katalysator durchgeführt wird, wobei das Synthesegas etwa 25 bis 60 Vol.-% CO, etwa 0 bis 4 Vol.-% $CO_2$, etwa 0 bis 4 Vol.-% $N_2$ bzw. $CH_4$ und als Rest $H_2$ enthält.

## Claims

1. A catalyst for the synthesis of alcohol mixtures containing methanol and higher alcohols from CO and $H_2$, which contains in the form of an oxide precursor,

a) copper oxide and zinc oxide which can be converted into the catalytically active component by reduction of at least one part of the copper oxide,

b) aluminium oxide as a thermostabilising substance and

c) at least one alkali carbonate or alkali oxide, characterised in that the oxide precursor has a percentage of pores with a diameter of between 14 and 7.5 nm of from 20 to 70 % of the total volume, the alkali content being approximately 13-$130 \times 10^{-6}$ gram atoms of alkali metal per gram of the oxide precursor and the aluminium oxide component having been

11

obtained from a colloidally dispersed aluminium hydroxide (aluminium hydroxide gel or sol) and the copper oxide and zinc oxide component (a) having been precipitated by the addition of an alkalinically reacting substance from a solution of the corresponding salts in the presence of the colloidally dispersed aluminium hydroxide.

2. A catalyst according to Claim 1, characterised in that the percentage of pores with a diameter of between 14 and 7.5 nm is 25 to 55 % of the entire pore volume.

3. A catalyst according to Claim 1 or 2, characterised in that the atomic ratio Cu/Zn is 1.3 to 3.8 :1, preferably 2.2 to 2.9 :1.

4. A catalyst according to any one of Claims 1 to 3, characterised in that it contains 5 to 25 % by weight, preferably 14 to 18 % by weight, of aluminium oxide (in relation to the oxide precursor).

5. A catalyst according to any one of Claims 1 to 4, characterised in that it contains $13\text{-}130 \times 10^{-6}$ gram atoms, preferably $50\text{-}100 \times 10^{-6}$ gram atoms, of potassium, rubidium and/or caesium per gram of the oxide precursor.

6. A catalyst according to any one of Claims 1 to 5, characterised in that as further thermostabilising substances it contains up to 10 % by weight, preferably 3 to 7 % by weight of $CeO_2$ and/or $La_2O_3$ (in relation to the oxide precursor).

7. A catalyst according to any one of Claims 1 to 6, which is obtained in that the precipitate containing the copper oxide and zinc oxide component (a) and the colloidally dispersed aluminium hydroxide are washed, optionally calcined, impregnated with compounds of the alkali metals, dried and compressed in known manner to form moulded bodies.

8. A catalyst according to Claim 1, which is obtained in that solutions of the nitrates of Cu and Zn and, optionally, of Ce and La are used and the precipitation is carried out with an aqueous $K_2CO_3$ solution having a concentration of preferably 5 to 20 % by weight.

9. A catalyst according to Claim 7 or 8, which is obtained in that the precipitation is carried out at temperatures of from 20 to 65 ºC and at pH values in the range of from 6.5 to 7.5.

10. A catalyst according to any one of Claims 7 to 9, which is obtained in that the precipitation is carried out by continuously bringing together the solution of the nitrates of Cu and Zn and, optionally, of Ce and La, and containing the colloidally dispersed aluminium hydroxide, with an aqueous $K_2CO_3$ solution.

11. A catalyst according to any one of Claims 7 to 10, which is obtained in that the washed precipitate of the oxide precursor is calcined at about 270 to 290 ºC, is optionally crushed and is rendered alkaline by being treated with an aqueous or aqueous-alcoholic solution of the alkali metal compound(s), preferably under reduced pressure.

12. A catalyst according to any one of Claims 7 to 11, which is obtained by using the hydroxides, carbonates, hydrogen carbonates, formates and/or acetates of potassium rubidium and/or caesium so as to render the oxide precursor alkaline.

13. Use of the catalyst according to any one of Claims 1 to 12 for the synthesis of alcohol mixtures containing methanol and higher alcohols from CO and $H_2$.

14. Use of the catalyst according to Claim 13, characterised in that the synthesis is carried out at a temperature of about 250 to 350 ºC, at a pressure of 80 to 150 bar and at a spatial velocity of about 1,000 to 10,000 litres of synthesis gas per hour per litre of catalyst, the synthesis gas containing about 25 to 60 % by volume of CO, about 0 to 4 % by volume of $CO_2$, about 0 to 4 % by volume of $N_2$ or $CH_4$ and $H_2$ as the remainder.

**Revendications**

1. Catalyseur pour la synthèse de mélanges d'alcools contenant du méthanol et des alcools supérieurs, à base de CO et de $H_2$, et qui, sous la forme d'un précurseur oxydique, contient :

a) de l'oxyde de cuivre et de l'oxyde de zinc pouvant être transformés en un composant à action catalytique par réduction d'au moins une partie de l'oxyde de cuivre,

b) de l'oxyde d'aluminium comme substance thermostabilisatrice,

c) au moins un carbonate alcalin ou un oxyde alcalin, caractérisé en ce que le précurseur oxydique possède une proportion de pores ayant un diamètre compris entre 14 et 7,5 nm de 20 à 70 % du volume total, en ce que la teneur atomes-grammes en alcali est approximativement de $13\text{-}130 \times 10^6$ de métal alcalin par gramme de précurseur oxydique, en ce que le composant d'oxyde d'aluminium a été obtenu à partir d'un hydroxyde d'aluminium à dispersion colloïdale (sol ou gel d'hydroxyde d'aluminium) et en ce que le composant (a) d'oxyde de cuivre et d'oxyde de zinc a été précipité par addition d'une substance à réaction basique provenant d'une solution des sels correspondants en présence de l'hydroxyde d'aluminium à dispersion colloïdale.

2. Catalyseur selon la revendication 1, caractérisé en ce que la proportion de pores ayant un diamètre compris entre 14 et 7,5 nm est comprise entre 25 et 55 % du volume total de pores.

3. Catalyseur selon l'une quelconque des revendications 1 et 2, caractérisé en ce que le rapport atomique Cu/Zn est compris entre 1,3 et 3,8 :1, et de façon préférentielle entre 2,2 et 2,9 :1.

4. Catalyseur selon l'une quelconque des revendications 1 à 3, caractérisé en ce qu'il contient entre 5 et 25 % en poids, et de façon préférentielle entre 14 et 18 % en poids d'oxyde d'aluminium (par rapport au précurseur oxydique).

5. Catalyseur selon l'une quelconque des revendications 1 à 4, caractérisé en ce qu'il contient 13-130 × 10⁶ atomes-grammes, et de façon préférentielle 50-100 × 10⁶ atomes-grammes de potassium, de rubidium et/ou de césium par gramme de précurseur oxydique.

6. Catalyseur selon l'une quelconque des revendications 1 à 6, caractérisé en ce qu'il contient, comme substances thermostabilisatrices supplémentaires, jusqu'à 10 % en poids, et de façon préférentielle entre 3 et 7 % en poids, de CeO₂ et ou de La₂O₃ (par rapport au précurseur oxydique).

7. Catalyseur selon l'une quelconque des revendications 1 à 6, caractérisé en ce qu'il est obtenu par lavage du précipité contenant le composant (a) d'oxyde de cuivre et d'oxyde de zinc, et l'hydroxyde d'aluminium à dispersion colloïdale, le cas échéant par calcination, par imprégnation avec des composés de métaux alcalins, par dessication, et par compression selon une méthode connue en soi.

8. Catalyseur selon la revendication 1, caractérisé en ce qu'il est obtenu par utilisation de solutions de nitrate de Cu et de Zn, ainsi que, le cas échéant, de nitrate de Ce et de La, et en ce qu'on réalise la précipitation avec une solution aqueuse de K₂CO₃ dont la concentration est comprise de façon préférentielle entre 5 et 20 % en poids.

9. Catalyseur selon l'une quelconque des revendications 7 et 8, caractérisé en ce qu'il est obtenu en réalisant la précipitation à une température comprise entre 20 et 65 °C et à une valeur pH comprise entre 6,5 et 7,5.

10. Catalyseur selon l'une quelconque des revendications 7 à 9, caractérisé en ce qu'il est obtenu en réalisant la précipitation par concentration continue de la solution de nitrate de Cu et de Zn, ainsi que le cas échéant de Ce et de La, avec une solution aqueuse de K₂CO₃, ladite solution de nitrate contenant l'hydroxyde d'aluminium à dispersion colloïdale.

11. Catalyseur selon l'une quelconque des revendications 7 à 10, caractérisé en ce qu'il est obtenu en calcinant le précipité lavé du précurseur oxydique à une température comprise approximativement entre 270 et 290 °C, le cas échéant en ce qu'on le concasse, et en ce qu'on l'alcalinise par traitement avec une solution aqueuse, ou une solution à base d'eau et d'alcools, des composés de métaux alcalins, et de préférence sous pression réduite.

12. Catalyseur selon l'une quelconque des revendications 7 à 11, caractérisé en ce qu'il est obtenu en utilisant, pour l'alcalinisation du précurseur oxydique, les hydroxydes, les carbonates, les hydrocarbonates, et les formiates et/ou acétate de potassium, de rubidium et/ou de césium.

13. Utilisation du catalyseur selon l'une quelconque des revendications 1 à 12 pour la synthèse de mélanges d'alcools contenant du méthanol et des alcools supérieurs, à base de CO et de H₂.

14. Utilisation selon la revendication 13, caractérisée en ce que la synthèse est réalisée à une température comprise approximativement entre 250 et 350 °C, sous une pression de 80 à 150 bars et à une vitesse spatiale comprise approximativement entre 1 000 et 10 000 litres de gaz de synthèse par heure et par litre de catalyseur, le gaz de synthèse contenant environ 25 à 60 % en volume de CO, environ 0 à 4 % en volume de CO₂, environ 0 à 4 % en volume de N₂, le cas échéant du CH₄, et du H₂ comme résidu.